# FASCICULE DE BREVET EUROPEEN

(11) **EP 1 680 418 B1**
(45) Date de publication et mention de la délivrance du brevet: **09.05.2007**
(21) Numéro de dépôt: 04791552.5
(22) Date de dépôt: 15.10.2004
(51) Int. Cl.: C07D 417/06

(54) **DERIVES DE N-[HETEROARYL(PIPERIDIN-2-YL)METHYL]BENZAMIDE, LEUR PREPARATION ET LEUR APPLICATION EN THERAPEUTIQUE**
DERIVATE VON N-[HETEROARYL(PIPERIDIN-2-YL)METHYL]BENZAMID, VERFAHREN ZU DEREN HERSTELLUNG UND DEREN ANWENDUNG IN THERAPEUTIKA
DERIVATIVES OF N-[HETEROARYL(PIPERIDINE-2-YL)METHYL]BENZAMIDE, PREPARATION METHOD THEREOF AND APPLICATION OF SAME IN THERAPEUTICS

(30) Priorité: 17.10.2003 FR 0312142
(43) Date de publication de la demande: 19.07.2006
(73) Titulaire: Sanofi-Aventis, 75013 Paris (FR)
(72) Inventeur: DARGAZANLI, Gihad, F-94230 Cachan (FR); ESTENNE-BOUHTOU, Geneviève, F-94550 Chevilly-Larue (FR); MEDAISKO, Florence, F-94100 Saint Maur des Fosses (FR); RENONES, Maria-Carmen, F-95880 Enghien-les-Bains (FR)
(74) Mandataire: Monain, Patrice
(86) Numéro de dépôt international: PCT/FR2004/002641
(87) Numéro de publication internationale: WO 2005/037781

(56) Documents cités:
- WO-A-01/81308

## Description

Les composés de l'invention répondent à la formule générale (I) 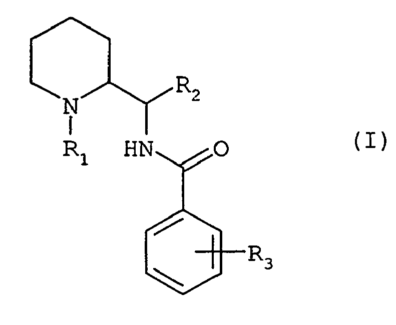 dans laquelle
R₁ représente soit un atome d'hydrogène, soit un groupe (C₁-C₇)alkyle linéaire ou ramifié éventuellement substitué par un ou plusieurs atomes de fluor, soit un groupe (C₃-C₇-cycloalkyle, soit un groupe (C₃-C₇) cycloalkyl (C₁-C₃) alkyle, soit un groupe phényl (C₁-C₃)alkyle éventuellement substitué par un ou deux groupes méthoxy, soit un groupe (C₂-C₉) alcényle, soit un groupe (C₂-C₄)alcynyle ;
R₂ représente un groupe pyridinyle, furanyle, thiényle, thiazolyle ou oxazolyle, ce groupe étant éventuellement substitué par un ou plusieurs substituants choisis parmi les atomes d'halogène et les groupes trifluorométhyle, (C₁-C₆) alkyle linéaire ou ramifié et (C₁-C₆) alcoxy ;
R₃ représente soit un atome d'hydrogène, soit un ou plusieurs substituants choisis parmi les atomes d'halogène et les groupes trifluorométhyle, (C₁-C₆)alkyle linéaire ou ramifié, (C₃-C₇)cycloalkyle, (C₁-C₆) alcoxy, phényle, cyano, acétyle, benzoyle, (C₁-C₆)thioalkyle, (C₁-C₆)alkylsulfonyle, carboxy ou (C₁-C₆) alcoxycarbonyle, soit un groupe de formule générale NR₄R₅ ou SO₂NR₄R₅ ou CONR₄R₅ dans lesquelles R₄ et R₅ représentent chacun, indépendamment l'un de l'autre, un atome d'hydrogène ou un groupe (C₁-C₆) alkyle linéaire ou ramifié ou (C₃-C₇) cycloalkyle, ou R₄ et R₅ forment, avec l'atome d'azote qui les porte, un cycle pyrrolidine, pipéridine ou morpholine.

Les composés de formule (I) peuvent comporter un ou plusieurs atomes de carbone asymétriques. Ils peuvent donc exister sous forme d'énantiomères ou de diastéréoisomères thréo ou érythro. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Les composés de l'invention présentent une activité particulière comme inhibiteurs spécifiques des transporteurs de la glycine glyt1 et/ou glyt2.

Les composés de formule générale (I), de configuration thréo ou érythro, dans laquelle R₁ est différent d'un atome d'hydrogène peuvent être préparés par un procédé illustré par le schéma 1 qui suit.

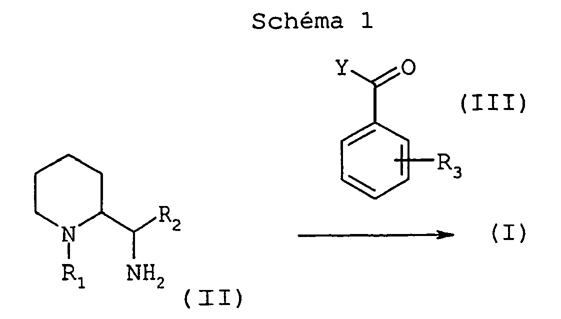

On effectue un couplage d'une diamine de formule générale (II), dans laquelle R₁ et R₂ sont tels que définis ci-dessus (avec R₁ différent d'un atome d'hydrogène) avec un acide activé ou un chlorure d'acide de formule générale (III) dans laquelle Y représente un groupe OH activé ou un atome de chlore et R₃ est tel que défini ci-dessus, en utilisant les méthodes connues de l'homme du métier.

La diamine de formule générale (II) peut être préparée par un procédé illustré par le schéma 2 qui suit.

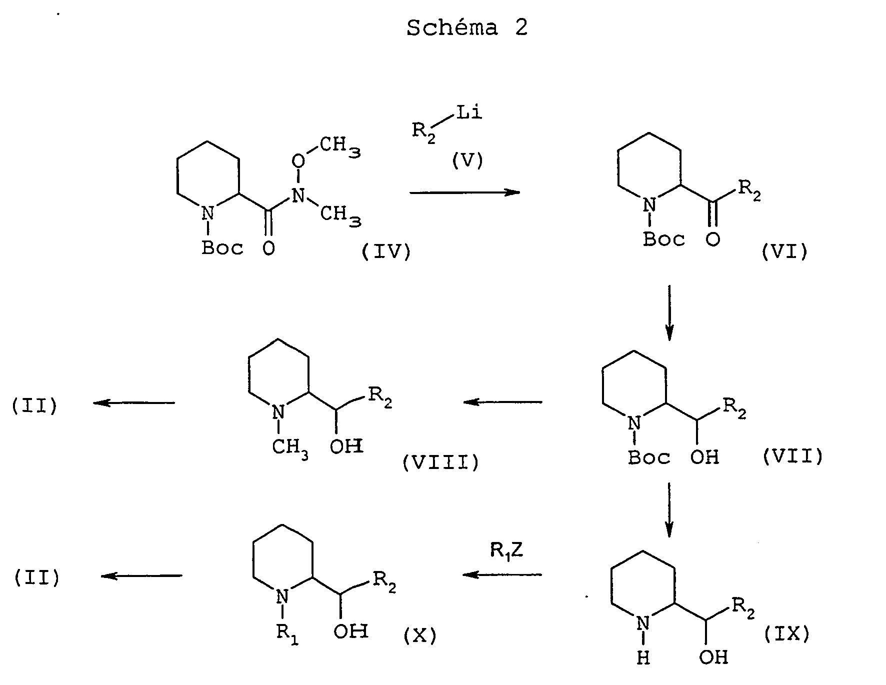

On fait réagir l'amide de Weinreb de formule (IV), dans laquelle Boc désigne un groupe 1,1-diméthyléthoxycarbonyle, avec l'hétérocycle lithié de formule générale (V), dans laquelle R₂ est tel que défini ci-dessus, dans un solvant éthéré tel que l'éther diéthylique, entre -90°C et -30°C ; on obtient une cétone de formule générale (VI) que l'on réduit en alcool de configuration thréo de formule générale (VII) par un agent réducteur tel que le K-Selectride® ou le L-Selectride® (tri-sec-butylborohydrure de potassium ou de lithium), dans un solvant éthéré tel que le tétrahydrofurane, entre -78°C et la température ambiante. Le carbamate de formule générale (VII) peut ensuite être réduit en N-méthylaminoalcool thréo de formule générale (VIII) par action d'un hydrure mixte tel que l'hydrure double d'aluminium et de lithium, dans un solvant éthéré tel que le tétrahydrofurane, entre la température ambiante et la température de reflux. On transforme ensuite en deux étapes l'alcool thréo de formule générale (VIII) en une diamine intermédiaire de formule générale (II) dans laquelle R₁ représente un groupe méthyle sous la forme thréo ou d'un mélange érythro-thréo dépendant de la nature de l'hétérocycle de la manière suivante : on transforme d'abord la fonction alcool en groupe nucléofuge, par exemple un groupe méthanesulfonate, par action du chlorure de méthylsulfonyle, dans un solvant chloré tel que le dichlorométhane, et en présence d'une base telle que la triéthylamine, entre 0°C et la température ambiante, puis on fait réagir le groupe nucléofuge avec de l'ammoniac liquéfié à -50°C, dans un alcool tel que l'éthanol, dans un milieu clos tel qu'un autoclave, entre -50°C et la température ambiante. On peut également déprotéger le carbamate de formule générale (VII) au moyen d'une base forte telle que la potasse aqueuse, dans un alcool tel que le méthanol pour obtenir l'aminoalcool thréo de formule générale (IX), procéder ensuite à une N-alkylation au moyen d'un dérivé halogéné de formule R₁Z, dans laquelle R₁ est tel que défini ci-dessus, mais différent d'un atome d'hydrogène, et Z représente un atome d'halogène, en présence d'une base telle que le carbonate de potassium, dans un solvant polaire tel que le *N,N*-diméthylformamide, entre la température ambiante et 100°C. On traite ensuite l'alcool de formule générale (X) ainsi obtenu comme décrit à propos de l'alcool de formule générale (VIII).

Les composés de formule générale (I) dans laquelle R₁ représente un atome d'hydrogène peuvent être préparés à partir d'un composé de formule générale (I) dans laquelle R₁ représente :
- soit un groupe phénylméthyle éventuellement substitué, en déprotégeant l'azote du cycle pipéridine, par exemple par un agent oxydant ou par un acide de Lewis tel que le tribromure de bore, ou par hydrogénolyse ,
- soit un groupe alcényle, de préférence allyle, en déprotégeant l'azote du cycle pipéridine, par exemple par un complexe du palladium 0,
pour obtenir un composé de formule générale (I) dans laquelle R₁ représente un atome d'hydrogène.

Par ailleurs les composés chiraux de formule générale (I) correspondants aux énantiomères *(1R,2R)-(1S,2S)-(1S,2R)* et *(1R,2S)* des différents diastéréoisomère erythro / thréo, peuvent être obtenus par séparation des composés racémiques par chromatographie liquide à haute performance (CLHP) sur colonne chirale, ou par dédoublement de l'amine racémique de formule générale (II) par utilisation d'un acide chiral, tel que l'acide tartrique, l'acide camphorsulfonique, l'acide dibenzoyltartrique, la N-acétylleucine, par la recristallisation fractionnée et préférentielle d'un sel diastéréoisomérique dans un solvant de type alcool, soit par synthèse énantiosélective selon le schéma 2 en utilisant un amide de Weinreb chiral de formule générale (IV).

L'amide de Weinreb de formule (IV) racémique ou chiral peut être préparé selon une méthode analogue à celle décrite dans *Eur. J. Med. Chem.,* **35,** (2000), 979-988 et *J. Med. Chem.,* **41**, (1998), 591-601. Les hétérocycles lithiés de formule générale (V) peuvent être préparés selon des méthodes connues de l'homme du métier et analogues à celles décrites dans *J.O.C.,* **62,** (1997), 5484-5496 et *Tetrahedron Letters,* **35**, (1994), 3673-3674.

Les dérivés halogénés de formule R₁Z sont disponibles dans le commerce .

Certains acides et chlorures d'acides de formule générale (III) sont disponibles dans le commerce ou, lorsqu'ils sont nouveaux, ils peuvent être obtenus selon des méthodes analogues à celles décrites dans les brevets EP-0556672, US-3801636, et dans *J. Chem. Soc.,* (1927), 25, *Chem. Pharm. Bull.,* (1992), 1789-1792, *Aust. J. Chem.,* (1984), 1938-1950 et *J*. *O*. *C*., (1980), 527.

Les exemples qui vont suivre illustrent la préparation de quelques composés de l'invention. Les microanalyses élémentaires, les spectres I.R. et R.M.N. et la CLHP sur colonne chirale confirment les structures et les puretés énantiométriques des composés obtenus.

Les numéros indiqués entre parenthèses dans les titres des exemples correspondent à ceux de la 1ère colonne du tableau donné plus loin.

Dans les noms des composés, le tiret "_" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°2). Chlorhydrate de

### 2-chloro-N-[(1-méthylpipér idin-2-yl)-3-thiénylméthyl]-3-trifluorométhylbenzamide 1:1.

### 1.1. 2-(3-thiénylcarbonyl)pipéridine-1-carboxylate de 1,1-diméthyléthyle.

Dans un ballon de 100 ml, sous atmosphère d'argon, on introduit 1,8 g (10,8 mmoles) de 3-bromothiophène en solution dans 20 ml d'éther diéthylique anhydre et on refroidit le milieu à -40°C. On ajoute ensuite lentement, 4,8 ml (12 mmoles) d'une solution 2,5M de butyllithium dans le cyclohexane et on laisse le mélange à cette température pendant 2 h.

A l'aide d'une aiguille de transfert, on ajoute l'hétérocycle lithié à une solution de 1,5 g (5,5 mmoles) de 2-(*N*-méthoxy-*N*-méthylcarbamoyl)pipéridine-1-carboxylate de 1,1-diméthyléthyle dans 50 ml d'éther diéthylique anhydre refroidie à -20°C et on laisse sous agitation le mélange revenir à température ambiante pendant 2 h.

Après hydrolyse avec une solution aqueuse saturée de chlorure d'ammonium, on sépare la phase aqueuse et on l'extrait avec de l'acétate d'éthyle. On sèche les phases organiques réunies sur sulfate de sodium, on filtre, on concentre sous pression réduite et on purifie le résidu par chromatographie sur colonne de gel de silice en éluant avec un mélange d'acétate d'éthyle et de cyclohexane.

On obtient 1,2 g d'un composée sous forme d'huile incolore que l'on utilise tel quel dans l'étape suivante.

### 1.2. thréo-[hydroxy(3 -thiényl)méthyl]pipéridine-1-carboxylate de 1,1-diméthyléthyle

Dans un ballon de 250 ml, sous atmosphère d'argon, on introduit 1,2 g (4 mmoles) de 2-(3-thiénylcarbonyl)-pipéridine-1-carboxylate de 1,1-diméthyléthyle dans 40 ml de tétrahydrofurane anhydre, on refroidit la solution à -78°C, on ajoute, goutte à goutte, 12 ml (12 mmoles) d'une solution 1M de L-Selectride® (tri-sec-butylborohydrure de lithium) dans le tétrahydrofurane et on agite le mélange à -78°C pendant 5 h.

On l'hydrolyse à froid lentement avec 7 ml d'eau et 7 ml d'une solution aqueuse à 35% de peroxyde d'hydrogène, et on laisse le mélange revenir à température ambiante en l'agitant pendant 2 h.

On le dilue avec de l'eau et de l'acétate d'éthyle, on sépare les phases et on extrait la phase aqueuse avec de l'acétate d'éthyle. Après lavage des phases organiques réunies, séchage sur sulfate de sodium, filtration et évaporation on purifie le résidu par chromatographie sur colonne de gel de silice en éluant avec un mélange d'acétate d'éthyle et de cyclohexane.

On obtient 1 g de produit sous forme d'huile incolore que l'on utilise tel quel dans l'étape suivante.

### 1.3. thréo-(1-méthylpipéridin-2-yl) (3-thiényl)méthanol.

Dans un bicol de 50 ml, sous atmosphère d'azote, on introduit 0,63 g (16,6 mmoles) d'hydrure double d'aluminium et de lithium dans 10 ml de tétrahydrofurane anhydre, on chauffe le mélange au reflux, on ajoute 1 g (3,3 mmoles) d'une solution de thréo-[hydroxy(3-thiényl)méthyl]-pipéridine-1-carboxylate de 1,1-diméthyléthyle dans 35 ml de tétrahydrofurane et on maintient le mélange au reflux pendant 2 h.

On le refroidit, on l'hydrolyse lentement avec une solution O,1M de tartrate double de potassium et de sodium et on laisse le mélange sous agitation pendant une nuit.

On le filtre et on rinçe le précipité avec du tétrahydrofurane, puis on concentre le filtrat sous pression réduite. On obtient 0,6 g d'un produit huileux incolore.

### 1.4. (1-méthylpipéridin-2-yl) (3-thiényl)méthanamine.

Dans un ballon de 50 ml, sous atmosphère d'azote, on introduit 0,6 g (2,8 mmoles) de thréo-(1-méthylpipéridin-2-yl) (3-thiényl)méthanol et O, 4 ml (2, 8 mmoles) de triéthylamine dans 10 ml de dichlorométhane anhydre, on refroidit le milieu à 0°C, on ajoute 0,22 ml (2,8 mmoles) de chlorure de méthanesulfonyle, on laisse le mélange revenir lentement à température ambiante pendant 1 h et on le concentre sous pression réduite. Dans un autoclave muni d'une agitation magnétique et refroidi à -50°C on introduit de l'ammoniac liquéfié, on ajoute une solution du méthanesulfonate brut précédemment préparé en solution dans 30 ml d'éthanol absolu, on ferme l'autoclave et on maintient l'agitation pendant 48 h.

On transvase le mélange dans un ballon, on concentre à sec, on dilue le résidu avec de l'eau et du dichlorométhane, on sépare les phases et on extrait la phase aqueuse avec du dichlorométhane. Après lavage des phases organiques réunies, séchage sur sulfate de sodium, filtration et évaporation des solvants, on isole 0,5 g d'amine sous forme de composé huileux que l'on utilise tel quel dans l'étape suivante.

### 1.5. Chlorhydrate de 2-chloro-N-[(1-méthylpipéridin-2-

yl) (3-thiényl)méthyl]-3-triflurométhylbenzamide 1:1 Dans un ballon de 50 ml on introduit, 0,25 g (1,17 mmoles) de (1-méthylpipéridin-2-yl) (3-thiényl)méthanamine et 0,26 ml (1,4 mmoles) de triethylamine en solution dans 20 ml de dichlorométhane à 0°C. On ajoute ensuite, une solution de 0,34 g (1,4 mmoles) de chlorure d'acide 2-chloro-3-triflorométhylbenzoïque dans 10 ml de dichlorométhane et on laisse le mélange sous agitation revenir à température ambiante pendant 2 h.

On traite le mélange avec de l'eau, on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol. On obtient 0,23 g de produit huileux qu'on isole sous forme de chlorhydrate à partir d'une solution 0,1N d'acide chlorhydrique dans le propan-2-ol.

On isole finalement 0,11 g de chlorhydrate sous forme de solide blanc composé d'un mélange de diastéréoisomères thréo / érythro dans un rapport 83/17.
Point de fusion : 124-126°C.

### Exemple 2 (Composé N°6).

### Chlorhydrate de thréo-2-chloro-3-méthyl-N-[(1-allylpipéridin-2-yl)-3-pyridinylméthyl]benzamide 1:1.

### 2.1. 2-(3-pyridinylcarbonyl)pipéridine-1-carboxylate de 1,1-diméthyléthyle

Dans un ballon de 500 ml, sous atmosphère d'argon, on introduit 14,5 g (91,8 mmoles) de 3-bromopyridine en solution dans 100 ml d'éther diéthylique anhydre et on refroidit le milieu à -78°C. On ajoute ensuite lentement, 40,4 ml (100,9 mmoles) d'une solution 2,5M de butyllithium dans le cyclohexane et on laisse le mélange à cette température pendant 0,5 h.

On ajoute une solution de 10 g (36,7 mmoles) de 2-(*N*-méthoxy-*N*-méthylcarbamoyl)pipéridine-1-carboxylate de 1,1-diméthyléthyle dans 50 ml d'éther diéthylique anhydre refroidie à -78°C et on laisse à cette température 2 heures sous agitation le mélange puis 12 heures à température ambiante.

Après hydrolyse avec une solution aqueuse saturée de chlorure d'ammonium, on sépare le phase aqueuse et on l'extrait avec de l'acétate d'éthyle. On sèche les phases organiques réunies sur sulfate de sodium, on filtre, on concentre sous pression réduite et on purifie le résidu par chromatographie sur colonne de gel de silice en éluant avec un mélange d'acétate d'éthyle et de cyclohexane.

On obtient 5,5 g d'un composée sous forme d'huile incolore que l'on utilise tel quel dans l'étape suivante.

### 2.2. thréo-[hydroxy(3-pyridinyl)méthyl]pipéridine-1-carboxylate de 1,1-diméthyléthyle.

Dans un ballon de 500 ml, sous atmosphère d'argon, on introduit 5,4 g (18,6 mmoles) de 2-(3-pyridinylcarbonyl)-pipéridine-1-carboxylate de 1,1-diméthyléthyle dans 220 ml de tétrahydrofurane anhydre, on refroidit la solution à -78°C, on ajoute, goutte à goutte, 55,8 ml (55,8 mmoles) d'une solution 1M de L-Selectride® (tri-*sec*-butylborohydrure de lithium) dans le tétrahydrofurane et on agite le mélange à -78°C pendant 3 h.

On l'hydrolyse à froid lentement avec 67 ml d'eau et 67 ml d'une solution aqueuse à 35% de peroxyde d'hydrogène, et on laisse le mélange revenir à température ambiante en l'agitant pendant 2 h.

On le dilue avec de l'eau et de l'acétate d'éthyle, on sépare les phases et on extrait la phase aqueuse avec de l'acétate d'éthyle. Après lavage des phases organiques réunies, séchage sur sulfate de sodium, filtration et évaporation, on purifie le résidu par chromatographie sur colonne de gel de silice en éluant avec un mélange d'acétate d'éthyle et de cyclohexane.

On obtient 4,13 g de produit sous forme d'huile incolore que l'on utilise tel quel dans l'étape suivante.

### 2.3. thréo-3-pyridinyl(2-pipéridin-2-yl)méthanol.

Dans un ballon de 50 ml on place une solution de 0,5 g (1,71 mmole) de thréo- [hydroxy(3-pryridinyl)méthyl]-pipéridine-1-carboxylate de 1,1-diméthyléthyle dans 6 ml d'éthanol, on ajoute une solution aqueuse de potasse préparée à partir de 0,5 g de potasse en pastilles et 3 ml d'eau, et on chauffe le mélange au reflux pendant 2 h.

On refroidit le mélange, on évapore le solvant sous pression réduite, on ajoute de l'eau et on extrait le mélange plusieurs fois avec du dichlorométhane. Après lavage des phases organiques réunies, séchage sur sulfate de magnésium, filtration et évaporation du solvant sous pression réduite, on obtient 0,3 g de solide blanc que l'on utilise tel quel dans l'étape suivante.

### 2.4. thréo-1-allylpipéridin-2-yl(3-pyridinyl)méthanol.

Dans un ballon de 50 ml muni d'une agitation magnétique et d'une circulation d'argon, on introduit 0,3 g (1,56 mmol) de thréo-3-pyridinyl(2-pipéridin-2-yl)méthanol et 10 ml d'acétonitrile. A la suspension obtenue on ajoute ensuite 0,32 g carbonate de potassium et 0,17 ml (1,2 eq) de bromure d'allyle. On maintient l'agitation de la suspension pendant 6h à 25°C. On ajoute 10 ml d'eau et 10 ml d'acétate d'éthyle, on décante et extrait la phase aqueuse trois fois avec 10 ml d'acétate d'éthyle, on lave les phases organiques réunies par 50 ml d'eau puis 500 ml d'une solution saturée de chlorure de sodium. On sèche sur sulfate de sodium, filtre et élimine les solvants sous pression réduite.

On obtient 0, 22 g d'huile jaune que l'on purifie par chromatographie sur gel de silice(colonne de 120 g et gradient d'élution de 2% à 10% de méthanol dans le dichlorométhane en 30 min). On isole 0,10 g sous forme d'huile jaune.

### 2.5. thréo-(1-allylpipéridin-2-yl) (3-pyridinyl)méthylamine.

Dans un ballon de 50 ml, sous atmosphère d'azote, on introduit 0, 71 g (3,05 mmoles) de thréo-1-allylpipéridin-2-yl (3-pyridinyl) méthanol et 0,43 ml (3,05 mmoles) de triéthylamine dans 15 ml de dichlorométhane anhydre, on refroidit le milieu à 0°C, on ajoute 0,23 ml (3,05 mmoles) de chlorure de méthanesulfonyle, on laisse le mélange revenir lentement à température ambiante pendant 1 h et on le concentre sous pression réduite.

Dans un autoclave muni d'une agitation magnétique et refroidi à - 50°C on introduit de l'ammoniac liquéfié, on ajoute une solution du méthanesulfonate brut précédemment préparé en solution dans 30 ml d'éthanol absolu, on ferme l'autoclave et on maintient l'agitation pendant 48 h.

On transvase le mélange dans un ballon, on concentre à sec, on dilue le résidu avec de l'eau et du dichlorométhane, on sépare les phases et on extrait la phase aqueuse avec du dichlorométhane. Après lavage des phases organiques réunies, séchage sur sulfate de sodium, filtration et évaporation on isole 0,57 g d'amine sous forme de composé huileux que l'on utilise tel quel dans l'étape suivante.

### 2.6. Chlorhydrate de thréo-2-chloro-3-méthyl-N-[(1-allylpipéridin-2-yl)-3-pyridinylméthyl]benzamide 1:1.

Dans un ballon de 50 ml on introduit successivement dans 10 ml de dichlorométhane, 0,22 g (1,28 mmole) d'acide 2,3-dichlorobenzoïque, 0,25 g (1,29 mmole) de chlorhydrate de 1-[3-(diméthylamino)propyl]-3-éthylcarbodiimide et 0,17 g (1,29 mmole)d'hydroxybenzotriazole et on agite le mélange à température ambiante pendant 1 h.

On ajoute 0,3 g (1,29 mmole) de thréo-(1-allylpipéridin-2-yl)(3-pyridinyl)méthylamine en solution dans 4 ml de dichlorométhane et on poursuit l'agitation pendant 15 h.

On traite le mélange avec de l'eau, on l'extrait plusieurs fois avec du dichlorométhane. Après lavage des phases organiques avec de l'eau puis avec une solution aqueuse de soude 1N, séchage sur sulfate de magnésium, filtration et évaporation du solvant sous pression réduite, on purifie le résidu par chromatographie sur colonne de gel de silice en éluant avec un mélange de dichlorométhane et de méthanol. On obtient 0,15 g de produit huileux qu'on isole sous forme de chlorhydrate à partir d'une solution 0,1N d'acide chlorhydrique dans le propan-2-ol.

On isole finalement 0,10 g de chlorhydrate sous forme de solide blanc.
Point de fusion : 149-151°C.

Le tableau qui suit illustre les structures chimiques et les propriétés physiques de quelques composés de l'invention.

Dans la colonne "Sel", "_" désigne un composé à l'état de base et "HCl" désigne un chlorhydrate.

**Tableau**

| | | | | | | |
|---|---|---|---|---|---|---|
| 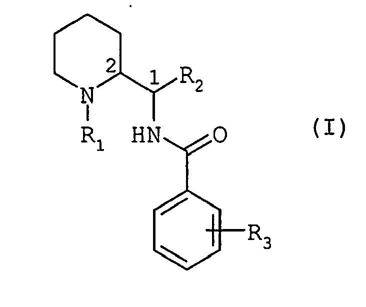 | | | | | | |

| N° | R₁ | R₂ | R₃ | Sel | F (°C) | Stéréochimie |
|---|---|---|---|---|---|---|
| 1 | CH₃ | 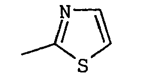 | 2-Cl, 3-CF₃ | HCl | 131-133 | Thréo/Erythro 0/100 |
| 2 | CH₃ | 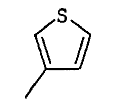 | 2-Cl, 3-CF₃ | HCl | 124-126 | Thréo/Erythro 83/17 |
| 3 | CH₃ | 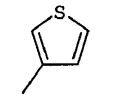 | 2-CH₃, 3-CF₃ | HCl | 139-141 | Thréo/Erythro 62/38 |
| 4 | CH₃ | 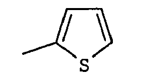 | 2- CH₃, 3-CF₃ | HCl | 166-168 | Thréo/Erythro 7/93 |
| 5 | CH₃ | 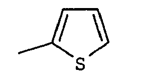 | 2-Cl, 3-CF₃ | HCl | 168-170 | Thréo/Erythro 0/100 |
| 6 | CH₂-CH=CH₂ | 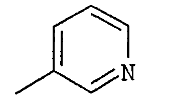 | 2-Cl, 3- CH₃ | HCl | 149-151 | Thréo/Erythro 100/0 |
| 7 | CH₂-CH=CH₂ | 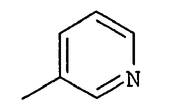 | 2-Cl, 3-CF₃ | HCl | 144-146 | Thréo/Erythro 100/0 |

Les composés de l'invention ont été soumis à une série d'essais pharmacologiques qui ont mis en évidence leur intérêt comme substances à activités thérapeutiques.

### Etude du transport de la glycine dans les cellules SK-N-MC exprimant le transporteur humain glyt1 natif.

La capture de [¹⁴C] glycine est étudiée dans les cellules SK-N-MC (cellules neuro-épithéliales humaines) exprimant le transporteur humain glyt1 natif par la mesure de la radioactivité incorporée en présence ou en absence du composé à tester. Les cellules sont cultivées en monocouche pendant 48 h dans des plaques prétraitées à la fibronectine à 0,02%. Le jour de l'expérience, le milieu de culture est éliminé et les cellules sont lavées par un tampon Krebs-HEPES (acide [4-(2-hydroxyéthyl)pipérazine-l-éthanesulfonique) à pH 7,4. Après 10 min de préincubation à 37°C en présence soit de tampon (lot témoin), soit de composé à tester a différentes concentrations ou de 10 mM de glycine (détermination de la capture non spécifique), 10 µM de [¹⁴C]glycine (activité spécifique 112 mCi/mmole) sont ensuite ajoutés. L'incubation se poursuit pendant 10 min à 37°C, et la réaction est arrêtée par 2 lavages avec un tampon Krebs-HEPES à pH 7,4. La radioactivité incorporée par les cellules est alors estimée après ajout de 100 µl de scintillant liquide et agitation pendant 1 h. Le comptage est réalisé sur compteur Microbeta Tri-lux^{™}. L'efficacité du composé est déterminée par la CI₅₀, concentration du composé qui diminue de 50% la capture spécifique de glycine, définie par la différence de radioactivité incorporée par le lot témoin et le lot qui a reçu la glycine à 10 mM.

Les composés les plus actifs de l'invention, dans ce test, ont une CI₅₀ de l'ordre de 0,001 à 1 µM.

### Etude du transport de la glycine dans l'homogénat de moelle épinière de souris.

La capture de [¹⁴C]glycine par le transporteur glyt2 est étudiée dans l'homogénat de moelle épinière de souris par la mesure de radioactivité incorporée en présence ou en absence de composé à étudier.

Après euthanasie des animaux (souris mâles OF1 Iffa Crédo pesant 20 à 25 g le jour de l'expérience), la moelle épinière de chaque animal est rapidement prélevée, pesée et conservée sur glace. Les échantillons sont homogénéisés dans un tampon Krebs-HEPES (acide [4-(2-hydroxyéthyl)pipérazine-1-éthanesulfonique), pH 7,4, à raison de 25 ml/g de tissu.

50 µl d'homogénat sont pré-incubés pendant 10 min à 25°C en présence de tampon Krebs-HEPES , pH 7,4 et de composé à étudier à différentes concentrations, ou de 10 mM de glycine pour déterminer la capture non spécifique. La [¹⁴C] glycine (activité spécifique = 112mCi/mmole) est ensuite ajoutée pendant 10 min à 25°C à la concentration finale de 10 µM. La réaction est arrêtée par filtration sous vide et la radioactivité est estimée par scintillation solide par comptage sur un compteur Microbeta Tri-lux^{™}. L'efficacité du composé est déterminée par la concentration CI₅₀ capable de diminuer de 50% la capture spécifique de glycine, définie par la différence de radioactivité incorporée par le lot témoin et le lot qui a reçu la glycine 10 mM.

Les composés les plus actifs de l'invention dans ce test, ont une CI₅₀ inférieure à 1 µM.

Il apparaît donc que les composés selon l'invention sont des inhibiteurs spécifiques des transporteurs de la glycine glyt1 et/ou glyt2.

Les composés selon l'invention peuvent donc être utilisés pour la préparation de médicaments, en particulier de médicaments inhibiteurs des transporteurs de la glycine glyt1 et/ou glyt2.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Les composés de l'invention peuvent être utilisés notamment pour le traitement des troubles comportementaux associés à la démence, des psychoses, en particulier de la schizophrénie (forme déficitaire et forme productive) et des symptômes extrapyramidaux aigus ou chroniques induits par les neuroleptiques, pour le traitement des diverses formes d'anxiété, des attaques de panique, des phobies, des troubles obsessionnels compulsifs, pour le traitement des différentes formes de dépression, y compris la dépression psychotique, pour le traitement des troubles dus à l'abus ou au sevrage d'alcool, des troubles du comportement sexuel, des troubles de la prise de nourriture, et pour le traitement de la migraine.

Ils peuvent également être utilisés pour le traitement des contractures musculaires douloureuses en rhumatologie et en pathologie rachidienne aiguë, pour le traitement des contractures spastiques d'origine médullaire ou cérébrale, pour le traitement symptomatique des douleurs aiguës et subaiguës d'intensité légère à modérée, pour le traitement des douleurs intenses et/ou chroniques, des douleurs neurogènes et algies rebelles, pour le traitement de la maladie de Parkinson et des symptômes parkinsoniens d'origine neurodégénérative ou induits par des neuroleptiques, pour le traitement des épilepsies généralisées primaires et secondaires, partielles à symptomatologie simple ou complexe, des formes mixtes et autres syndromes épileptiques en complément d'un autre traitement antiépileptique, ou en monothérapie, pour le traitement des apnées du sommeil, et pour la neuroprotection.

La présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec un ou plusieurs excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également l'utilisation d'un composé selon l'invention pour la préparation d'un médicament destiné au traitement des pathologies ci-dessus indiquées ledit traitement comprenant l'administration, à un patient, d'une dose efficace d'un composé selon l'invention, ou un de ses sels pharmaceutiquement acceptables ou hydrates ou solvats.

## Revendications

1. Composé répondant à la formule générale (I) 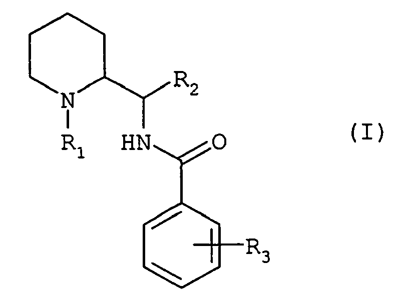 dans laquelle
R₁ représente soit un atome d'hydrogène, soit un groupe (C₁-C₇)alkyle linéaire ou ramifié éventuellement substitué par un ou plusieurs atomes de fluor, soit un groupe (C₃-C₇-cycloalkyle, soit un groupe (C₃-C₇)cycloalkyl(C₁-C₃)alkyle, soit un groupe phényl(C₁-C₃)alkyle éventuellement substitué par un ou deux groupes méthoxy, soit un groupe (C₂-C₄)alcényle, soit un groupe (C₂-C₄) alcynyle ;
R₂ représente un groupe pyridinyle, furanyle, thiényle, thiazolyle ou oxazolyle, ce groupe étant éventuellement substitué par un ou plusieurs substituants choisis parmi les atomes d'halogène et les groupes trifluorométhyle, (C₁-C₆) alkyle linéaire ou ramifié et (C₁-C₆) alcoxy
R₃ représente soit un atome d'hydrogène, soit un ou plusieurs substituants choisis parmi les atomes d'halogène et les groupes trifluorométhyle, (C₁-C₆)alkyle linéaire ou ramifié, (C₃-C₇)cycloalkyle, (C₁-C₆) alcoxy, phényle, cyano, acétyle, benzoyle, (C₁-C₆)thioalkyle, (C₁-C₆)alkylsulfonyle, carboxy ou (C₁-C₆)alcoxycarbonyle, soit un groupe de formule générale NR₄R₅ ou SO₂NR₄R₅ ou CONR₄R₅ dans lesquelles R₄ et R₅ représentent chacun, indépendamment l'un de l'autre, un atome d'hydrogène ou un.groupe (C₁-C₆) alkyle linéaire ou ramifié ou (C₃-C₇) cycloalkyle, ou R₄ et R₅ forment, avec l'atome d'azote qui les porte, un cycle pyrrolidine, pipéridine ou morpholine ;
à l'état de base libre ou de sel d'addition à un acide, d'hydrate ou de solvat.

2. Médicament **caractérisé en ce qu'**il comprend un composé selon la revendication 1, ou un sel d'addition de ce composé à un acide pharmaceutiquement acceptable ou encore un hydrate ou un solvat du composé de formule (I).

3. Composition pharmaceutique, **caractérisée en ce qu'**elle comprend un composé selon la revendication 1, ou un sel pharmaceutiquement acceptable, un hydrate ou un solvat de ce composé, ainsi qu'au moins un excipient pharmaceutiquement acceptable.

4. Utilisation d'un composé de formule (I) selon la revendication 1 pour la préparation d'un médicament destiné au traitement des troubles comportementaux associés à la démence, des psychoses, des diverses formes d'anxiété, des attaques de panique, des phobies, des troubles obsessionnels compulsifs, des différentes formes de dépression, des troubles dus à l'abus ou au sevrage d'alcool, des troubles du comportement sexuel, des troubles de la prise de nourriture et de la migraine.

5. Utilisation d'un composé de formule (I) selon la revendication 1 pour la préparation d'un médicament destiné au traitement des contractures, de la douleur, de la maladie de Parkinson et des symptômes parkinsoniens, des épilepsies, des formes mixtes et autres syndromes épileptiques en complément d'un autre traitement antiépileptique, ou en monothérapie, des apnées du sommeil, et pour la neuroprotection.

## Claims

1. Compound corresponding to the general formula (I) 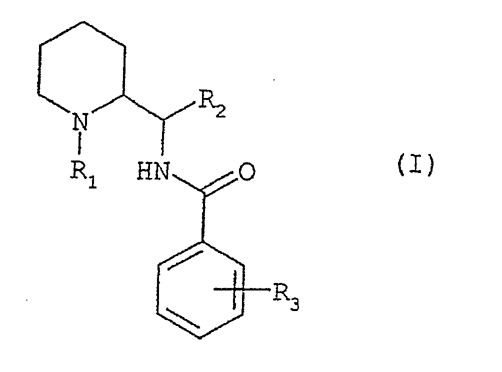 in which
R₁ represents either a hydrogen atom, or a linear or branched (C₁-C₇) alkyl group optionally substituted with one or more fluorine atoms, or a (C₃-C₇)cycloalkyl group, or a (C₃-C₇)cycloalkyl(C₁-C₃)alkyl group, or a phenyl(C₁-C₃)alkyl group optionally substituted with one or two methoxy groups, or a (C₂-C₄)alkenyl group, or a (C₂-C₄) alkynyl group;
R₂ represents a pyridyl, furyl, thienyl, thiazolyl or oxazolyl group, this group being optionally substituted with one or more substituents chosen from halogen atoms and trifluoromethyl and linear or branched (C₁-C₆) alkyl and (C₁-C₆) alkoxy groups;
R₃ represents either a hydrogen atom, or one or more substituents chosen from halogen atoms and trifluoromethyl, linear or branched (C₁-C₆) alkyl, (C₃-C₇)cycloalkyl, (C₁-C₆)alkoxy, phenyl, cyano, acetyl, benzoyl, (C₁-C₆) thioalkyl, (C₁-C₆) alkylsulfonyl, carboxyl or (C₁-C₆)alkoxycarbonyl groups, or a group of general formula NR₄R₅ or SO₂NR₄R₅ or CONR₄R₅ in which R₄ and R₅ each independently represent a hydrogen atom or a linear or branched (C₁-C₆) alkyl or (C₃-C₇) cycloalkyl group, or R₄ and R₅ form, with the nitrogen atom that bears them, a pyrrolidine, piperidine or morpholine ring;
in the form of free base or of acid-addition salt, hydrate or solvate.

2. Medicament, **characterized in that** it comprises a compound according to Claim 1, or an addition salt of this compound with a pharmaceutically acceptable acid or alternatively a hydrate or a solvate of the compound of formula (I).

3. Pharmaceutical composition, **characterized in that** it comprises a compound according to Claim 1, or a pharmaceutically acceptable salt, a hydrate or a solvate of this compound, and also at least one pharmaceutically acceptable excipient.

4. Use of a compound of formula (I) according to Claim 1, for the preparation of a medicament for treating behavioral disorders associated with dementia, psychoses, various forms of anxiety, panic attacks, phobias, compulsive obsessive disorders, various forms of depression, disorders caused by alcohol abuse or weaning from alcohol, sexual behavior disorders, eating disorders and migraine.

5. Use of a compound of formula (I) according to Claim 1, for the preparation of a medicament for treating contractures, pain, Parkinson's disease and Parkinson-like symptoms, epilepsy, mixed forms and other epileptic syndromes in addition to another antiepileptic treatment, or in monotherapy, and sleep apnea, and for neuroprotection.

## Patentansprüche

1. Verbindung der allgemeinen Formel (I) 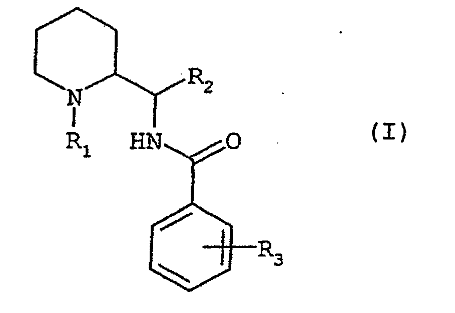 worin
R₁ für ein Wasserstoffatom, eine gegebenenfalls durch ein oder mehrere Fluoratome substituierte lineare oder verzweigte (C₁-C₇) -Alkylgruppe, eine (C₃-C₇)-Cycloalkylgruppe, eine (C₃-C₇) -Cycloalkyl-(C₁-C₃)-alkylgruppe, eine gegebenenfalls durch eine oder zwei Methoxygruppen substituierte Phenyl-(C₁-C₃) -alkylgruppe, eine (C₂-C₄) -Alkenylgruppe oder eine (C₂-C₄)-Alkinylgruppe steht;
R₂ für eine Pyridinyl-, Furanyl-, Thienyl-, Thiazolyl- oder Oxazolylgruppe steht, die gegebenenfalls durch einen oder mehrere unter Halogenatomen, Trifluormethyl-, linearen oder verzweigten (C₁-C₆)-Alkyl- und (C₁-C₆)-Alkoxygruppen ausgewählte Substituenten substituiert ist;
R₃ für ein Wasserstoffatom, einen oder mehrere unter Halogenatomen und Trifluormethyl-, linearen oder verzweigten (C₁-C₆)-Alkyl-, (C₃-C₇)-Cycloalkyl-, (C₁-C₆)-Alkoxy-, Phenyl-, Cyano-, Acetyl-, Benzoyl-, (C₁-C₆) -Thioalkyl-, (C₁-C₆) -Alkylsulfonyl-, Carboxy- oder (C₁-C₆)-Alkoxycarbonylgruppen ausgewählte Substituenten oder eine Gruppe der allgemeinen Formel NR₄R₅, SO₂NR₄R₅ oder CONR₄R₅, worin R₄ und R₅ jeweils unabhängig voneinander ein Wasserstoffatom oder eine lineare oder verzweigte (C₁-C₆)-Alkyl- oder eine (C₃-C₇) -Cycloalkylgruppe bedeuten oder mit dem Stickstoffatom, an das sie gebunden sind, einen Pyrrolidin-, Piperidin- oder Morpholinring bilden, steht;
in Form der freien Base oder in Form eines Säureadditionssalzes, Hydrats oder Solvats.

2. Medikament, **dadurch gekennzeichnet, daß** es eine Verbindung nach Anspruch 1 oder ein Additionssalz dieser Verbindung mit einer pharmazeutisch unbedenklichen Säure oder auch ein Hydrat oder ein Solvat der Verbindung der Formel (I) enthält.

3. Pharmazeutische Zusammensetzung, **dadurch gekennzeichnet, daß** sie eine Verbindung nach Anspruch 1 oder ein pharmazeutisch unbedenkliches Salz, ein Hydrat oder ein Solvat dieser Verbindung sowie mindestens einen pharmazeutisch unbedenklichen Trägerstoff enthält.

4. Verwendung einer Verbindung der Formel (I) nach Anspruch 1 zur Herstellung eines Medikaments zur Behandlung von Verhaltensstörungen in Assoziation mit Demenz, Psychosen, verschiedenen Formen von Angst, Panikattacken, Phobien, Zwangsneurosen, verschiedenen Formen von Depression, Störungen, die auf Alkoholmißbrauch oder -entzug zurückzuführen sind, Störungen des Sexualverhaltens, Störungen der Nahrungsaufnahme und Migräne.

5. Verwendung einer Verbindung der Formel (I) nach Anspruch 1 zur Herstellung eines Medikaments zur Behandlung von Kontrakturen, Schmerz, Parkinson-Krankheit und Parkinson-ähnlichen Symptomen, Epilepsien, Mischformen und anderen epileptischen Syndromen zusätzlich zu einer anderen antiepileptischen Behandlung oder bei der Monotherapie und Schlafapnoe und zur Neuroprotektion.
